# FASCICULE DE BREVET EUROPEEN

(11) **EP 0 770 140 B1**
(45) Date de publication et mention de la délivrance du brevet: **18.07.2007**
(21) Numéro de dépôt: 95925026.7
(22) Date de dépôt: 07.07.1995
(51) Int. Cl.: C12N 15/87, C07H 21/00, A61K 47/48

(54) **COMPOSITION CONTENANT DES ACIDES NUCLEIQUES ET DES POLYMERES CATIONIQUES, PREPARATION ET UTILISATION**
ZUSAMMENSETZUNG ENTHALTEND NUKLEINSÄUREN UND KATIONISCHE POLYMERE, ZUBEREITUNG UND VERWENDUNG
COMPOSITION CONTAINING NUCLEIC ACIDS AND CATIONIC POLYMERS, PREPARATION AND USES

(30) Priorité: 13.07.1994 FR 9408735
(43) Date de publication de la demande: 02.05.1997
(73) Titulaire: CENTELION, 94400 Vitry Sur Seine (FR)
(72) Inventeur: BEHR, Jean-Paul, F-67100 Strasbourg (FR); BOUSSIF, Otmane, F-67100 Strasbourg (FR); DEMENEIX, Barbara, F-75013 Paris (FR); LEZOUALCH, Franck, F-29100 Douarnenez (FR); MERGNY, Mojgan, F-94800 Villejuif (FR); SCHERMAN, Daniel, F-75013 Paris (FR)
(74) Mandataire: Texier, Christian
(86) Numéro de dépôt international: PCT/FR1995/000914
(87) Numéro de publication internationale: WO 1996/002655

(56) Documents cités:
- EP-A- 0 424 688
- WO-A-91/16024
- WO-A-93/05162
- WO-A-93/20090
- WO-A-94/01448
- WO-A-94/05624

## Description

La présente invention concerne des compositions à base d'acides nucléiques, leur préparation et leur utilisation. Plus particulièrement, elle concerne des compositions comprenant au moins un acide nucléique et certains polymères cationiques, et leur utilisation pour le transfert d'acides nucléiques dans les cellules, notamment en thérapie génique.

La thérapie génique consiste à corriger une déficience ou une anomalie (mutation, expression aberrante, etc) ou à assurer l'expression d'une protéine d'intérêt thérapeutique par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. Différentes techniques ont été décrites pour le transfert de cette information génétique, parmi lesquelles des techniques diverses de transfection impliquant des complexes d'ADN et de DEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), d'ADN et de polylysine, l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physicochimiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

Toutefois, les techniques développées jusqu'à présent ne permettent pas de résoudre de manière satisfaisante les difficultés liées au transfert de gènes dans les cellules et/ou l'organisme. En particulier, les problèmes liés à la pénétration de l'acide nucléique dans les cellules ne sont pas entièrement résolus. En effet, la nature polyanionique des acides nucléiques, notamment, prévient leur passage à travers les membranes cellulaires. S'il a été montré que les acides nucléiques nus sont capables de traverser la membrane plasmique de certains types cellulaires in vivo (voir notamment la demande n° WO90/11092), l'efficacité de transfection reste assez faible. De plus, les acides nucléique nus ont une demi-vie plasmatique courte, en raison de leur dégradation par les enzymes et de leur élimination par les voies urinaires. Par ailleurs, si les virus recombinants permettent d'améliorer l'efficacité de transfert des acides nucléiques, leur emploi présente certains risques tels que la pathogénicité, la transmission, la réplication, la recombinaison, la transformation, l'immunogénicité, etc. De plus, leur production selon les normes de Bonne Pratique de fabrication présente certaines difficultés.

La demande de brevet WO A 93 20090 divulgue des conjugués polymères cationiques-oligonucléotides couplés entre eux par liaison covalente au moyen d'un agent de couplage, le rapport cations sur anions desdits conjugués étant compris entre 0,8 et 2.

La demande de brevet EPA 0424688 divulgue une méthode pour modifier des cellules végétales afin d'incorporer un ADN étranger. Cette méthode comprend une étape de prétraitement des cellules avec un polycation, puis le traitement de ladite cellule prétraitée avec une suspension comprenant de l'acide nucléique et des liposomes cationiques.

La présente invention apporte une solution avantageuse à ces différents problèmes. La demanderesse a en effet montré que certains polymères cationiques possèdent des propriétés particulièrement avantageuses pour le transfert d'acides nucléiques dans les cellules, in vitro comme in vivo. En outre, ces polymères présentent l'avantage d'être facilement accessibles et peu couteux. L'utilisation des polymères cationiques selon l'invention permet également d'éviter les inconvénients liés à l'emploi de vecteurs viraux (dangers potentiels, taille limitée du gène transféré, prix élevé, etc).

Plus particulièrement, la présente invention réside dans la mise en évidence des propriétés transfectantes des polymères de formule (I) : 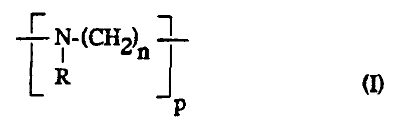 dans laquelle
- R peut être un atome d'hydrogène ou un groupe de formule 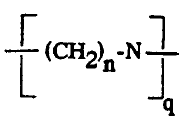
- n est un nombre entier compris entre 2 et 10;
- p et q sont des nombres entiers,
étant entendu que la somme p+q est telle que le poids moléculaire moyen du polymère soit compris entre 100 et 10⁷ Da, et les proportions respectives du polymère et de l'acide nucléique sont choisies de sorte que le rapport R amines du polymère / phosphates de l'acide nucléique soit compris autre 5 et 30.

Il est entendu que, dans la formule (I) la valeur de n peut varier entre les différents motifs p. Ainsi, la formule (I) regroupe à la fois les homopolymères et les hétéropolymères.

Un premier objet de l'invention réside donc dans une composition constitué d' un acide nucléique et d'une solution d'un polymère cationique réunies et homogénéisées, le polymère cationique étant de formule générale (I) telle que définie ci-dessus.

L'invention concerne également l'utilisation des polymères cationiques de formule (I) pour le transfert d'acides nucléiques dans les cellules.

Plus préférentiellement, dans la formule (I), n est compris entre 2 et 5. En particulier, les polymères de polyéthylène imine (PEI) et polypropylène imine (PPI) présentent des propriétés tout à fait avantageuses.

Les polymères préférés pour la mise en oeuvre de la présente invention sont ceux dont le poids moléculaire est compris entre 10³ et 5.10⁶ Da. A titre d'exemple, on peut citer le polyéthylène imine de poids moléculaire moyen 50 000 Da (PEI50K) ou le polyéthylène imine de poids moléculaire moyen 800 000 Da (PEI800K).

Les polymères utilisés dans le cadre de la présente invention peuvent être obtenus de différentes manières. Ils peuvent tout d'abord être synthétisés chimiquement à partir du monomère correspondant, en conditions de polymérisation anionique (par exemple polymérisation de l'éthylène imine), ou par réduction de polyamides obtenus par polycondensation de diacides avec des diamines, ou encore par réduction d'imines obtenues par polycondensation de dialdéhydes avec des diamines. Par ailleurs, un certain nombre de ces polymères sont accessibles commercialement, tels que notamment le PEI50K ou le PEI800K.

Pour obtenir un effet optimum des compositions de l'invention, les proportions respectives du polymère et de l'acide nucléique sont de préférence déterminées de sorte que le rapport molaire R = amines du polymère / phosphates de l'acide nucléique soit compris entre 0,5 et 50; plus préférentiellement entre 5 et 30. Des résultats tout particulièrement avantageux sont obtenus en utilisant de 5 à 15-équivalents d'amines de polymère par charge d'acide nucléique. Ce rapport peut bien entendu être adapté par l'homme du métier en fonction du polymère utilisé, de la présence d'un adjuvant (voir ci-après), de l'acide nucléique, de la cellule cible et du mode d'administration utilisé.

Dans les compositions de la présente invention, l'acide nucléique peut être aussi bien un acide désoxyribonucléique qu'un acide ribonucléique. Il peut s'agir de séquences d'origine naturelle ou artificielle, et notamment d'ADN génomique, d'ADNc, d'ARNm, d'ARNt, d'ARNr, de séquences hybrides ou de séquences synthétiques ou semi-synthétiques. En outre, l'acide nucléique peut avoir une taille très variable, allant de l'oligonucléotide au chromosome. Ces acides nucléiques peuvent être d'origine humaine, animale, végétale, bactérienne, virale, etc. Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent par ailleurs être incorporés dans des vecteurs, tels que des vecteurs plasmidiques.

Concernant plus particulièrement les acides désoxyribonucléiques, ils peuvent être simple ou double brin. Ces acides désoxyribonucléiques peuvent porter des gènes thérapeutiques, des séquences régulatrices de la transcription ou de la réplication, des séquences antisens, des régions de liaison à d'autres composants cellulaires, etc.

Au sens de l'invention, on entend par gène thérapeutique notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier à une expression insuffisante dans la cellule ou à l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire. Le gène thérapeutique peut également coder pour une protéine sécrétée dans l'organisme.

Parmi les produits thérapeutiques au sens de la présente invention, on peut citer plus particulièrement les enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, HARP/pléiotrophine, etc; les apolipoprotéines : ApoAI, ApoAIV, ApoE, etc (FR 9305125), la dystrophine ou une minidystrophine (FR 9111947), la protéine CFTR associée à la mucoviscidose, les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, les gènes intervenant dans la réparation de l'ADN, les gènes suicides (thymidine kinase, cytosine déaminase), etc.

Le gène thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Il peut s'agir aussi d'oligonucléotides synthétiques, éventuellement modifiés (EP 92 574). Les antisens comprennent également les séquences codant pour des ribozymes, qui sont capables de détruire sélectivement des ARN cibles (EP 321201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, ou encore spécifiques de tumeurs (EP 259 212).

Préférentiellement, l'acide nucléique comprend également des séquences permettant l'expression du gène thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc. En outre, ces séquences d'expression peuvent être modifiées par addition de séquences d'activation, de régulation, ou permettant une expression tissu-spécifque.

Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

Par ailleurs encore, l'acide nucléique peut également comporter, en particulier en amont du gène thérapeutique, une séquence dirigeant le produit thérapeutique synthétisé vers un compartiment cellulaire préférentiel, comme une séquence de localisation nucléaire.

Les compositions selon l'invention peuvent être utilisées telles quelles ou en association avec d'autres composés. Ainsi, dans un mode particulier de mise en oeuvre, les compositions selon la présente invention comprenent en plus un adjuvant capable de s'associer au complexe polymère/acide nucléique et d'améliorer le pouvoir transfectant, ledit adjuvant étant choisi parmi les lipides, protéines, lipopolyamines et polymères synthétiques capables de s'associer au complexe polymère/acide nucléique. Comme le montrent les exemples de la présente demande, cette amélioration se manifeste aussi bien in vitro que in vivo.

Les adjuvants utilisés préférentiellement dans les compositions selon l'invention sont des lipides cationiques (comportant une ou plusieurs charges cationiques dans leur partie polaire) ou des lipides neutres.

Concernant les lipides cationiques, il peut s'agir plus particulièrement de lipopolyamines, c'est-à-dire de toute molécule amphiphile comprenant au moins une région hydrophile polyamine et une région lipophile liées covalamment entre-elles par un bras chimique. Avantageusement, la région polyamine des lipopolyamines utilisées dans le cadre de l'invention répond à la formule générale H₂N-(-(CH)ₘ-NH-)₁-H dans laquelle m est un nombre entier supérieur ou égal à 2 et 1 est un nombre entier supérieur ou égal à 1, m pouvant varier entre les différents groupes de carbone compris entre deux amines. Préférentiellement, m est compris entre 2 et 6 inclusivement et 1 est compris entre 1 et 5 inclusivement. Encore plus préférentiellement, la région polyamine est représentée par la spermine ou un analogue de la spermine ayant conservé ses propriétés de liaison à l'ADN.

La région lipophile peut être une ou plusieurs chaînes hydrocarbonées, saturées ou non, du cholestérol, un lipide naturel ou un lipide synthétique capables de former des phases lamellaires ou hexagonales.

Avantageusement, on utilise dans le cadre de la présente invention des lipopolyamines telles que définies dans la demande de brevet EP 394 111. Cette demande décrit également un procédé utilisable pour la préparation de ces lipopolyamines. De manière particulièrement avantageuse, on utilise dans le cadre de l'invention la dioctadécylamidoglycyl spermine (DOGS) ou la 5-carboxyspermylamide de la palmitoylphosphatidylethanolamine (DPPFS).

D'autres adjuvants particulièrement préférés pour la réalisation des compositions de l'invention sont représentés par les lipides neutres. L'utilisation de lipides neutres est particulièrement avantageuses lorsque le rapport de charges R (amines/phosphates) est faible. Plus préférentiellement, les lipides neutres utilisés dans le cadre de la présente invention sont des lipides à 2 chaines grasses.

De manière particulièrement avantageuse, on utilise des lipides naturels ou synthétiques, zwitterioniques ou dépourvus de charge ionique dans les conditions physiologiques. Il peuvent être choisis plus particulièrement parmi la dioleoylphosphatidyléthanolamine (DOPE), le oléoyl-palmitoylphosphatidyléthanolamine (POPE), le di-stéaroyl, -palmitoyl, -mirystoyl phosphatidyléthanolamine ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM1 et GM2).

Ces différents lipides peuvent être obtenus soit par synthèse, soit par extraction à partir d'organes (exemple : le cerveau) ou d'oeufs, par des techniques classiques bien connues de l'homme du métier. En particulier, l'extraction des lipides naturels peut être réalisée au moyen de solvants organiques (voir également Lehninger, Biochemistry).

Préférentiellement, les compositions de l'invention comprennent, en plus du polymère cationique dans les rapports cités ci-avant, de 0,1 à 20 équivalents molaires d'adjuvant pour 1 équivalent molaire de phosphate de l'acide nucléique, et, plus préférentiellement, de 1 à 5.

Dans un mode de réalisation particulièrement avantageux, les compositions de la présente invention comprennent un élément de ciblage permettant d'orienter le transfert de l'acide nucléique. Cet élément de ciblage peut être un élément de ciblage extracellulaire, permettant d'orienter le transfert de l'acide nucléique vers certains types cellulaires ou certains tissus souhaités (cellules tumorales, cellules hépatiques, cellules hématopoiétiques, etc). Il peut également s'agir d'un élément de ciblage intracellulaire, permettant d'orienter le transfert de l'acide nucléique vers certains compartiments cellulaires privilégiés (mitochondries, noyau, etc).

Plus préférentiellement, l'élément de ciblage est lié, de manière covalente ou non covalente, au polymère de formule (I). La liaison peut notamment être obtenue par interaction ionique avec les ammonium, ou par attaque nucléophile des amines du polymère sur des éléments de ciblage comportant un groupement nucléofuge (halogène, tosylate, etc), un ester activé (hydroxysuccinimide, etc) ou encore un isothiocyanate. L'élément de ciblage peut également être lié à l'acide nucléique.

Parmi les éléments de ciblage utilisables dans le cadre de l'invention, on peut citer les sucres, les peptides, les oligonucléotides ou les lipides. Préférentiellement, il s'agit de sucres et/ou peptides tels que des anticorps ou fragments d'anticorps, des ligands de récepteurs cellulaires ou des fragments de ceux-ci, des récepteurs ou fragments de récepteurs, etc. En particulier, il peut s'agir de ligands de récepteurs de facteurs de croissance, de récepteurs de cytokines, de récepteurs de lectines cellulaires ou de récepteurs de protéines d'adhésion. On peut également citer le récepteur de la transférine, des HDL et des LDL. L'élément de ciblage peut également être un sucre permettant de cibler les récepteurs asialoglycoprotéiques, ou encore un fragment Fab d'anticorps permettant de cibler le récepteur du fragment Fc des immunoglobulines.

Les compositions selon l'invention peuvent être formulées en vue d'administrations par voie topique, cutanée, orale, rectale, vaginale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée.

Comme indiqué ci-avant, les compositions selon l'invention peuvent être utilisées pour le transfert d'acides nucléiques dans les cellules in vivo, in vitro ou ex vivo. En particulier, les exemples montrent que les polymères cationiques de l'invention peuvent être utilisés pour transférer de manière très efficace des acides nucléiques dans de nombreux types cellulaires, et en particulier dans certains types cellulaires habituellement difficiles à transfecter. Parmi les types cellulaires testés on peut citer notamment les fibroblastes, les cellules hépatiques, les carcinomes, les cellules rénales et les neurones. En outre, les exemples présentés ci-après illustrent également l'efficacité des polymères de l'invention pour le transfert de gènes in vivo. Les résultats obtenus avec les vecteurs de l'invention sont supérieurs à ceux observés dans les mêmes conditions avec d'autres agents de transfection, et démontrent ainsi les potentialités élevées des compositions de l'invention.

La demande décrit une méthode particulièrement avantageuse pour le traitement de maladies, comprenant l'administration in vivo d'un acide nucléique apte à corriger ladite maladie associé à un polymère cationique tel que défini ci-avant. Plus particulièrement, cette méthode est applicable aux maladies résultant d'une déficience en un produit protéique ou nucléique et l'acide nucléique administré code pour ledit produit protéique ou contient ledit produit nucléique.

Les compositions pharmaceutiques de l'invention constituent ainsi des outils particulièrement avantageux pour l'administration et le transfert d'acides nucléiques in vivo.

La présente invention sera plus complètement décrite à l'aide des exemples qui suivent, qui doivent être considérés comme illustratifs.

### Légende des figures :

Figure 1 : Efficacité de transfection du PEI800K à pH7 en fonction du rapport R (amines du polymère / phosphates de l'acide nucléique).
Figure 2 : Efficacité de transfection du PEI800K à pH6 en fonction de la quantité d'ADN.
Figure 3 : Efficacité de transfection du PEI800K à pH6 en fonction du rapport R.
Figure 4 : Efficacité de transfection du PEI800K en fonction du pH
Figure 5 : Comparaison entre le PEI800K et la polylysine
Figure 6 : Test de cytotoxicité du PEI50K
Figure 7 : Efficacité de transfection du PEI50K en présence d'adjuvants
Figure 8 : Efficacité du PEI pour la transfection des cellules HeLa
Figure 9 : Efficacité du PEI pour la transfection des cellules hépatiques et rénales.
Figure 10 : Efficacité du PEI pour le transfert d'ADN plasmidique dans les neurones embryonnaires
Figure 11 : Efficacité du PEI pour le transfert d'oligonucléotides dans les neurones embryonnaires
Figure 12 : Efficacité du PEI pour le transfert d'ADN in vivo

### EXEMPLE 1 - Plasmides utilisés pour le transfert de gènes in vivo

Trois types de constructions ont été utilisées pour mettre en évidence l'activité des compositions de l'invention : Des plasmides comportant le gène codant pour la luciférase (Luc), des plasmides comportant le gène codant pour la β-galactosidase (gène LacZ) et des plasmides comportant le gène codant pour la chloramphénicol acétyl transférase (CAT).

### 1.1. Plasmides comportant le gène Luc

Le plasmide pCMV-luc comporte le promoteur du Cytomégalovirus (CMV), extrait du plasmide vecteur pcDNA3 (Invitrogen) par coupure avec les enzymes de restriction Mlu I et HindIII, situé en amont du gène codant pour la luciférase, inséré aux sites MluI et HindIII dans le vecteur pGL basic Vector (Promega).

Le plasmide pGL2-Luc est d'origine commerciale (Promega).

Le plasmide T3RE-Luc contient un oligonucléotide synthétique correspondant à un élément de réponse palindromique à l'hormone thyroïdienne (Glass et al., Cell 56 (1989) 697).

### 1.2. Plasmides comportant le gène LacZ

Le plasmide pCMV-βGal (Clontech) comporte le promoteur CMV situé en amont du gène LacZ codant pour la β-galactosidase d'Escherichia coli. Le vecteur pSV-nls LacZ (pAOnlsLacZ) comporte le même promoteur, une séquence de localisation nucléaire (issue du virus SV40) localisée en phase et en amont du gène LacZ. Cette construction permet l'expression de la protéine de fusion nls-β-galactosidase dans le noyau des cellules (Cf De Luze et al., PNAS 90 (1993) 7322).

### 1.3. Plasmides comportant le gène CAT

Les plasmides ayant pour gène rapporteur le gène codant pour la chloramphénicol acétyl transférase (CAT) sous contrôle des promoteurs RSV (pRSV-CAT) et SV40 (pSV40-CAT) ont été publiés (Boutiller et al., Prog. Neuro-PhychoPharmacol. et Biol. Psychiat. 16 (1992) 959; de Luze et al. PNAS 90 (1993) 7322).

### EXEMPLE 2 - Transfert d'acide nucléique dans les fibroblastes.

Cet exemple décrit le transfert du plasmide pGL2-luc dans des fibroblastes 3T3 au moyen de polyéthylène imine 800 000 Da.

Protocole : Une solution 5,375 µM de PEI 800 K est préparée et le pH est ajusté à 7 grâce à une solution d'acide chlorhydrique 1 N. Cette solution est utilisée pour les expériences de transfert de gènes.

Protocole de transfection : Les fibroblastes 3T3 sont ensemencées dans une boite 24 puits 24 heures avant la transfection (50,000 cellules par puits). Elles sont transfectées avec 2 µg de plasmide pGL2-Luc par puits selon le protocole suivant :

2 µg de plasmide et différents volumes de la solution 5,375 µM de PEI 800 K (en fonction du rapport d'équivalent de charges souhaité) sont dilués séparément dans 50 µl de Nacl 150 mM et mélangés. Après 10 minutes, les deux solutions sont réunies et homogéneisées. Après une nouvelle période de 10 minutes, 900 µl de DMEM sont ajoutés. La solution ainsi obtenue est homogénéisée et après 10 minutes, elle est répartie sur les cellules préalablement rincées avec du milieu DMEM sans sérum. Au bout de 2 heures de transfection. 100 µl de SVF (sérum de veau foetal) est rajouté par puits. L'expression est arrêtée 24 heures après.

Dosage de la luciférase. Pour cela, le surnageant a été incubé en présence d'un tampon comprenant de la luciférine, du coenzyme A et de l'ATP, et la lumière émise (généralement pendant 10 secondes) a été mesurée avec un luminomètre (Wood K. (1990) Promega Notes, 28).

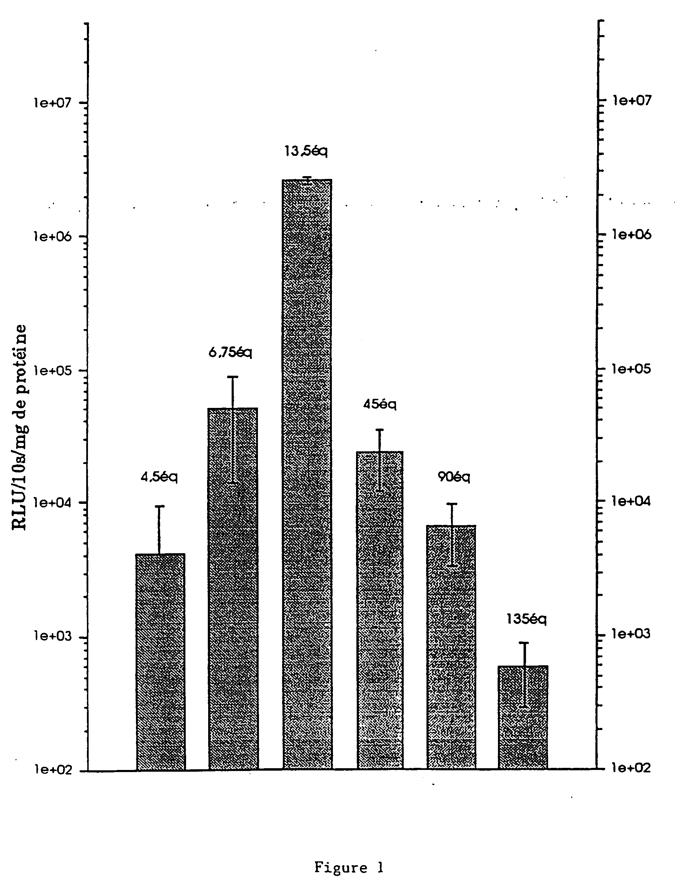

Les résultats obtenus sont présentés sur la figure 1. Ils montrent que le PEI permet de transférer efficacement le plasmide pGL2-Luc dans les fibroblastes. Ils montrent également que l'activité en unités lumière relative (RLU = "relative light unit") est particulièrement bonne lorsque l'on utilise entre 5 et 20 équivalents d'amines du PEI par rapport aux phosphates de l'ADN. Dans une expérience contrôle, le plasmide seul donne un signal de 100 RLU environ.

### EXEMPLE 3 - Effet de la quantité d'ADN sur l'efficacité de la transfection

Cet exemple décrit l'effet de la quantité d'acide nucléique sur l'efficacité du transfert dans les fibroblastes 3T3 par le PEI800K.

Les fibroblastes 3T3 sont ensemencées dans une boîte 24 puits 24 heures avant la transfection (50, 000 cellules par puits). Elles sont transfectées avec des quantités croissantes de plasmide pCMV-Luc par puits et un rapport de charges constant (9 équivalents) selon le protocole décrit préalablement. Deux points ont été réalisés en complétant la quantité de plasmide à deux µg par puits par du plasmide pGEM (PROMEGA) ne contenant pas le gène étudié. Au bout de 3 heures de transfection. 100 µl de sérum SVF est rajouté par puits. L'expression est arrêtée 24 heures après.

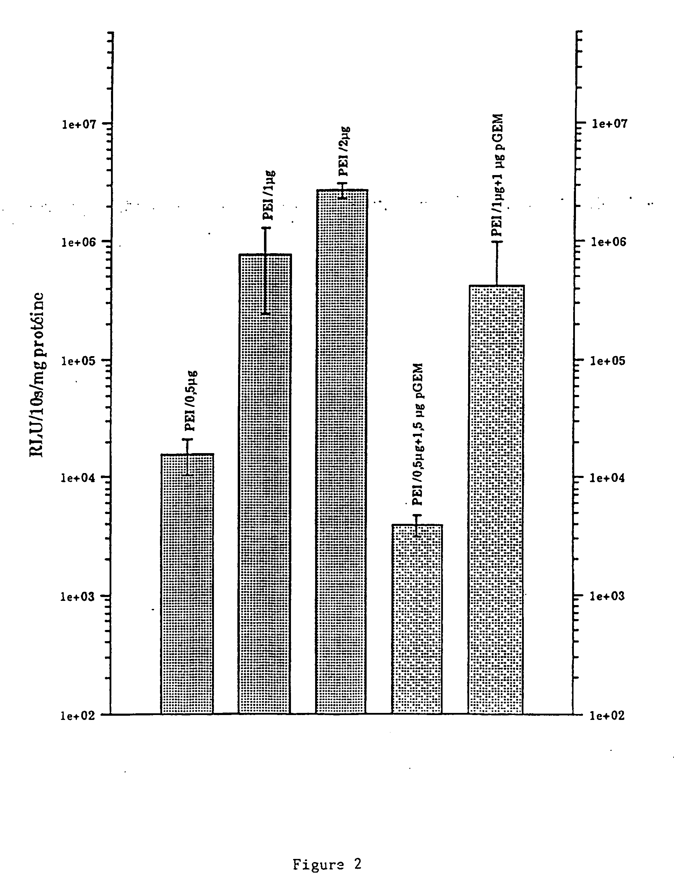

Les résultats obtenus sont représentés sur la figure 2.

L'efficacité de transfection croît avec la quantité de plasmide utilisée lors de la transfection. L'addition d'ADN porteur non transcrit (pGEM) n'a pas d'apport pour la transfection.

### EXEMPLE 4 - Transfert d'acides nucléiques dans les fibroblastes : Etude de l'influence du rapport aminés/phosphates.

Cet exemple décrit le transfert d'acides nucléiques dans les fibroblastes à pH 6 au moyen d'une composition selon l'invention comprenant l'acide nucléique et le PEI800K dans différents rapports R (amines/phosphates).

Protocole : Une solution 5,375 µM de PEI 800 K est préparée et le pH est ajusté à 6 grâce à une solution d'acide chlorhydrique 1 N. Cette solution est utilisée pour les expériences de transfert de gènes.

Les fibroblastes 3T3 sont ensemencées dans une boîte 24 puits 18 heures avant la transfection (50,000 cellules par puits). Elles sont transfectées avec 2µg de plasmide pGL2-Luc par puits selon le protocole utilisé dans l'exemple 2.

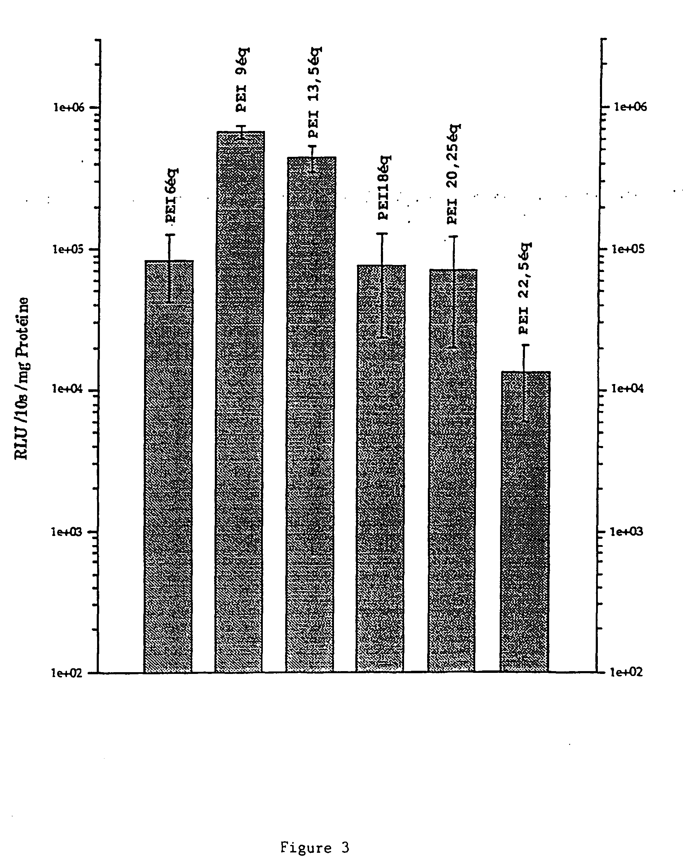

Les résultats obtenus sont présentés sur la figure 3. Ils montrent que les compositions selon l'invention sont particulièrement efficaces dans toute une gamme de rapports R comprise entre 6 et 22.

### EXEMPLE 5 - Efficacité de transfection en fonction du pH

Cet exemple décrit le transfert d'acides nucléiques dans les fibroblastes au moyen d'une composition selon l'invention comprenant l'acide nucléique et le PEI 800K, dans différentes conditions de pH.

Le protocole utilisé est identique à celui décrit dans l'exemple 2. Le pH est ajusté par addition d'acide chlorhydrique 1N.

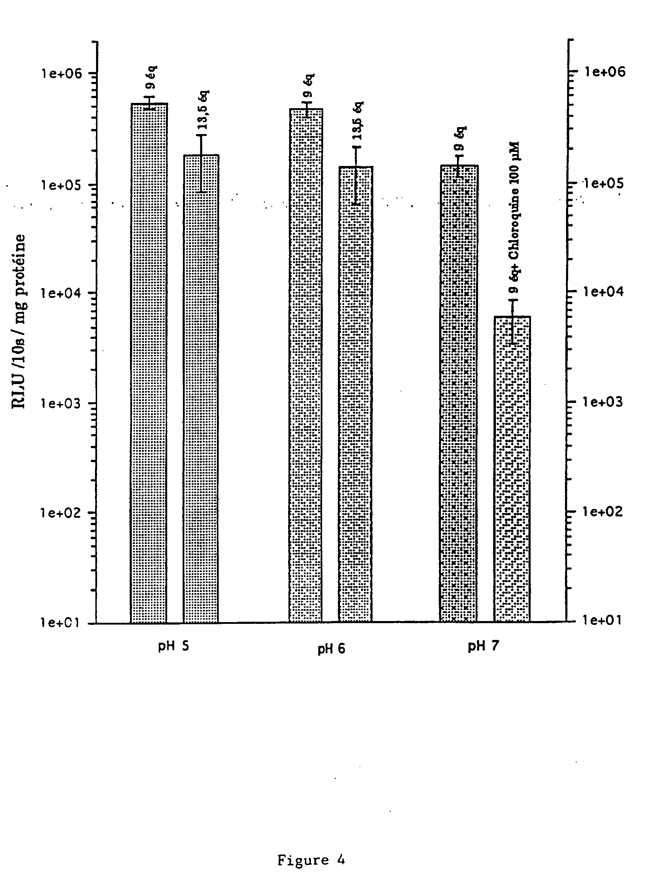

Les résultats obtenus sont présentés sur la figure 4. Ils montrent que les compositions selon l'invention peuvent être utilisées à différents pH et notamment dans des zones de pH physiologique (pH 5 -8).

### EXEMPLE 6 - Comparaison de l'efficacité de transfection du PEI et de la polylysine

Cet exemple décrit les résultats comparatifs obtenus avec une composition selon l'invention et avec un autre polymère cationique, la polylysine, pour le transfert d'acides nucléiques dans les fibroblastes.

Protocole : La polylysine (PLL) utilisée dans cette expérience est de la polylysine, HBr de poids moyen 50 K. La concentration finale en chloroquine est 100 µM. Le PEI est à pH 6.

Protocole : Les fibroblastes 3T3 sont ensemencées dans une boîte 24 puits 18 heures avant la transfection (50,000 cellules par puits). Elles sont transfectées avec du plasmide pCMV-Luc selon le protocole utilisé dans l'exemple 2. Les cellules transfectées avec la PLL en présence de chloroquine sont rincées au bout de 2 heures et mises dans du milieu avec 10 % de sérum. L'expression est arrêtée en 24 heures après.

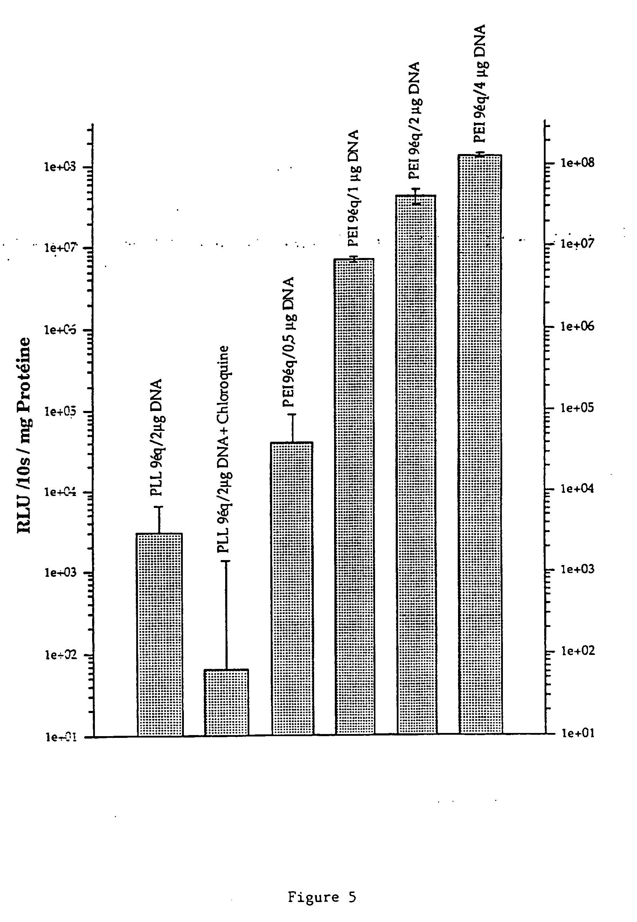

Les résultats obtenus sont présentés sur la figure 5. Ils démontrent clairement que les compositions selon l'invention permettent de transférer des acides nucléiques dans les fibroblastes avec une efficacité bien supérieure aux vecteurs polymériques cationiques antérieurs tels que la polylysine.

### EXEMPLE 7 - Comparaison de l'efficacité de transfection des Fibroblastes NIH 3T3 par le PEI 50K et le PEI 800K

Cet exemple décrit l'utilisation et la comparaison de deux polymères de PEI (PEI 50K et PEI 800K), pour le transfert d'acides nucléiques dans les cellules.

Protocole : Les cellules sont ensemencées 24 heures avant la transfection à 50 000 cellules par puits de 16 mm. Elles sont transfectées avec le plasmide pCMV Luc complexé avec le PEI. Le rapport d'équivalent molaire amine de PEI/phosphate d'acide nucléique varie de 6 à 45 équivalents. Pour chaque puit, on dilue séparément le PEI et 2 µg d'ADN dans 50 µl NaCl (150 mM). Les solutions sont ensuite mélangées, puis homogénisées. Après 10 minutes, ce volume est ajouté aux cellules. Après 24 heures, les cellules sont lysées, la solution de lyse centrifugée, et le surnageant utilisé pour doser l'activité luciférase. Le dosage de protéine est effectué sur un aliquot de ce surnageant par le test de BCA. L'activité luciférase est exprimée en unité de lumière (RLU)/10 secondes d'intégration/mg de protéine.

Les résultats obtenus sont présentés dans le tableau ci-dessous.

| R | O | 6éq | 9éq | 18éq | 45éq |
|---|---|---|---|---|---|
| **PEI 50K RLU/10s/mg Prot** | 7 100 | 11 000 | 760 000 | 12 000 000 | 2 450 000 |
| **PEI 800K RLU/10s/mgProt** | 6 253 | 200 000 | 16 700 000 | 5 650 000 | 525 000 |

Ces résultats indiquent clairement que le PEI 50K possède des propriétés transfectantes aussi avantageuses que le PEI 800K.

### EXEMPLE 8 - Test de cytotoxicité du PEI

Nous avons voulu étudier la toxicité éventuelle du PEI 50K sur des fibroblastes NIH3T3. Pour cette étude, nous avons choisi le test MTT qui permet d'évaluer la capacité des cellules à réduire le MTT tétrazolium, en MTT formazan, par l'enzyme mitochondriale, succinate deshydrogénase. Le protocole de transfection est le même que dans l'exemple 7 (la quantité d'ADN par puits reste fixe (2 µg), le nombre d'équivalent molaire amine de PEI/phospate de l'ADN varie de 9 à 24). Après 24 heures de transfection, les cellules sont lavées trois fois avec du PBS, puis incubées pendant 90 minutes avec 0,05 mg/ml de MTT. Au terme de cette incubation, le milieu des cellules est éliminé, les cellules sont lavées trois fois avec du PBS, puis dissoutes dans 1 ml de SDS 10 %, pendant 10 minutes. La densité optique des échantillons est lue à 540 nm.

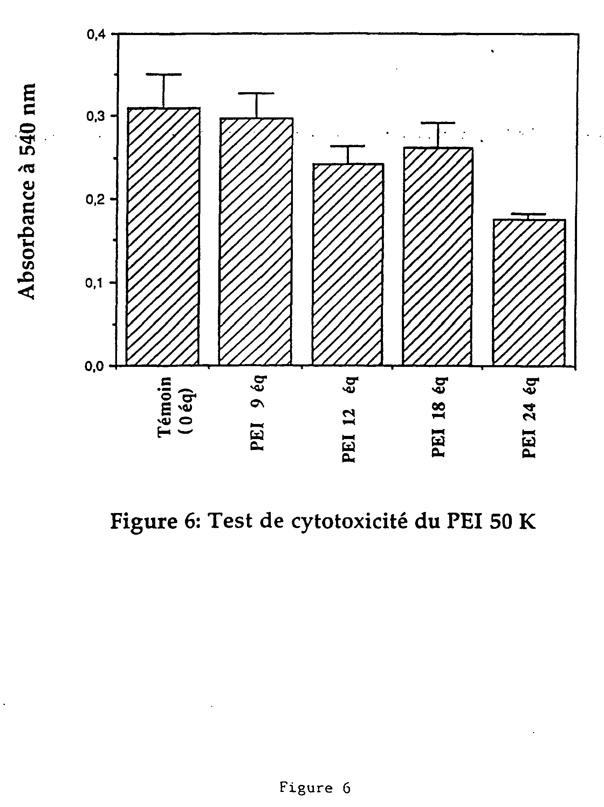

Les résultats sont présentés sur la figure 6. Ils montrent que dans les conditions de notre étude le PEI 50K n'entraine pas de mortalité significative pour les fibroblastes NIH 3T3.

### EXEMPLE 9 - Utilisation d'un adjuvant pour améliorer le pouvoir transfectant

Cet exemple décrit l'utilisation de compositions selon l'invention comprenant un polymère cationique et un adjuvant. L'adjuvant utilisé est soit un lipide neutre (la DOPE); soit une lipopolyamine (le DOGS).

### Protocole : Ajout de la DOPE :

Quatre rapports équivalents molaires d'amine de PEI/phosphate d'ADN ont été étudiés : 6, 9, 18 et 21. Pour chaque puit, 12 nanomoles de DOPE sont ajoutés au mélange PEI et ADN. Cette solution est ensuite utilisée pour la transfection comme décrit dans l'exemple 7.

Ajout du DOGS : Pour chaque puit, 4 équivalents molaires d'amine de DOGS/phosphate d'ADN (soit 6 nanomoles de DOGS pour 6 nanomoles de phosphates) sont ajoutés à la solution PEI avant l'étape de complexation avec l'ADN. La transfection est effectuée ensuite comme décrit dans l'exemple 7.

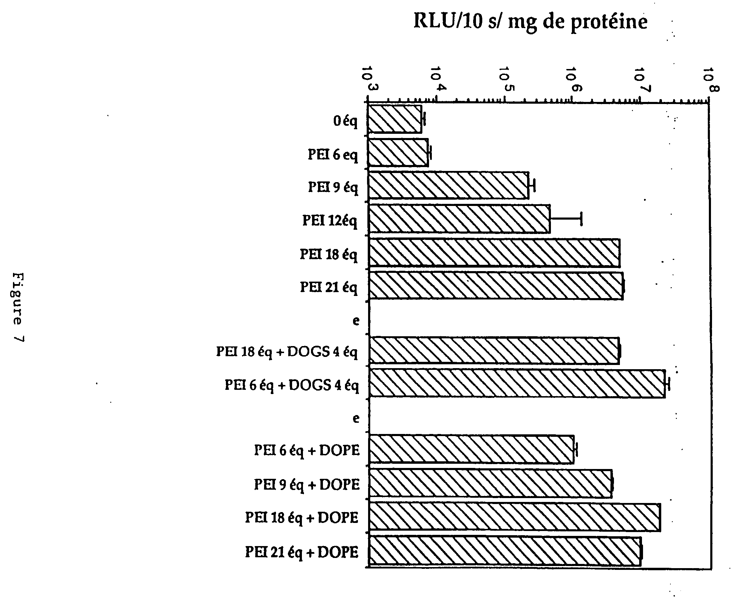

Les résultats obtenus sont présentés sur la figure 7. Ils montrent clairement que l'ajout de la DOPE ou du DOGS permet d'augmenter encore les propriétées de transfection, notamment pour des rapports d'équivalents molaires d'amine de PEI/phosphats d'ADN faibles.

### EXEMPLE 10 - Transfert de gène dans les Cellules Hela

Cet exemple démontre que les compositions selon l'invention peuvent être utilisées pour le transfert de gènes dans différents types cellulaires, et notamment dans les cellules de carcinome utérin Hela.

Protocole : Les cellules Hela sont ensemmencées 24 heures avant la transfection à 50,000 cellules par puits de 16 mm. Elles sont ensuite transfectées avec le plasmide pCMV Luc complexé soit avec le PEI soit avec la lipopolyamine (DOGS). Quatre rapports d'équivalents molaires d'amine de PEI 50K ou de PEI 800K/phosphate d'ADN ont été étudié : 6, 9, 18,21. La lipopolyamine est utilisé à 8 équivalents molaires d'amine/phosphate (12 nanonomoles de DOGS).

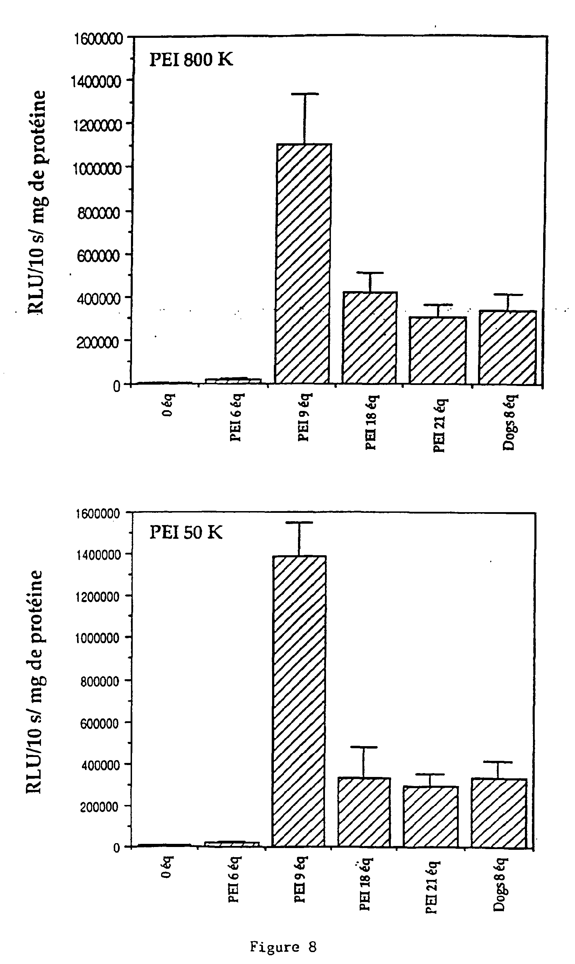

Les résultats obtenus sont présentés sur la figure 8. Ils montrent clairement que les compositions selon l'invention comprenant un polymère cationique éventuellement associé à un adjuvant, permettent de transfecter les cellules Hela de manière beaucoup plus efficace que les systèmes antérieurs (la lipopolyamine, DOGS, notamment).

### EXEMPLE 11 - Transfert de gènes dans différents types cellulaires.

Cet exemple montre encore que les compositions selon l'invention peuvent être utilisées pour le transfert de gènes dans des types cellulaires très variés, tels que les fibroblastes, les carcinomes utérins, les hépatocytomes ou des cellules de rein. Plus particulièrement, cet exemple décrit la transfection des cellules HepG2, K 562 et de cultures primaires de muscle lisse de lapin par le PEI à 9 équivalents de charges et àpH6.

Les cellules HepG 2 sont ensemencées dans des boîtes 24 puits 24 heures avant la transfection (50,000 cellule par puits). Elles sont transfectées avec le plasmide pCMV-Luc selon le protocole décrit dans l'exemple 2. Au bout de 4 heures de transfection,100 µl de sérum SVF est rajouté par puits. L'expression est arrêtée 30 heures après.

Les cellules K 562 sont ensemencées dans des boîtes 24 puits, dans 0,5 ml de milieu RPMI sans sérum une heure avant transfection. Elles sont transfectées avec du plasmide pCMV-Luc selon le protocole suivant : La quantité de plasmide désirée et le PEI sont dilués séparément dans 50 µl de Nacl 150 mM et mélangés. Après 10 minutes, les deux solutions sont réunies et homogéneisées. Après une nouvelle période de 10 minutes, 400 µl de RPMI sont ajoutés. La solution ainsi obtenue est homogénéisée et après 10 minutes elle est répartie sur les cellules. Au bout de 4 heures de transfection, 100 µl de sérum SVF est rajouté par puits. L'expression est arrêtée 30 heures après.

Les cultures primaires de muscle lisse de lapin ont été préparées comme décrit par Fallier-Becker. Les cellules ont ensuite été transfectées soit avec 1 µg soit avec 2 µg de plasmide pCMV-Luc complexé avec le PEI (selon le protocole décrit dans l'exemple 7). L'expression de la luciférase est arrêtée 24 ou 48 heures après la transfection.

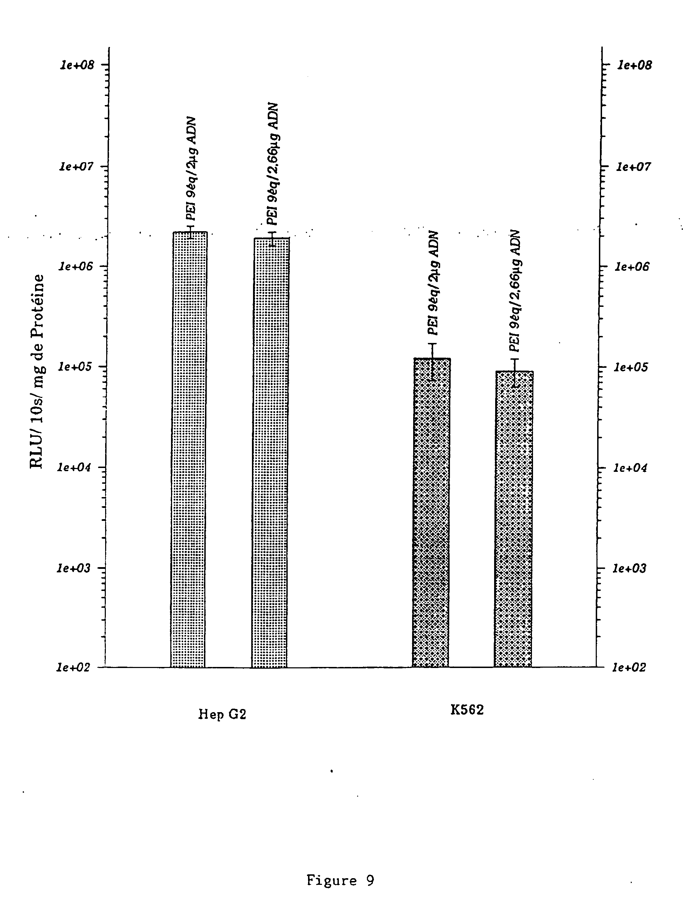

Les résultats obtenus sont présentés dans la figure 9. Ils montrent clairement que les compositions selon l'invention permettent la transfection efficace de types cellulaires très variés et différents.

### EXEMPLE 12 - Transfert d'ADN plasmidique dans une culture primaire de neurones embryonaires : démonstration d'une activité biologique.

Les cultures primaires de neurones ont été préparées comme décrit par Lezoualc'h et al. Après 36 h, les cultures ont été transfectées avec 2 µg de plasmide TRE-Luc (comportant le gène Luc sous contrôle d'un élément de réponse à l'hormone thyroïdienne T3) et 0,5 µg d'ADN carrier par puits.

Deux conditions expérimentales ont été étudiées:
- 9 équivalents d'amines par rapport aux phosphates : 1µg de plasmide a été introduit dans 2,28 µl d'une solution aqueuse de PEI800K 100mM, pH7.
- 13 équivalents d'amines par rapport aux phosphates : 1µg de plasmide a été introduit dans 3,33 µl d'une solution aqueuse de PEI800K 100mM, pH7.

Préalablement à la complexation, le plasmide a été dilué dans 50µl de NaCl 150mM, et le PEI a aussi été dilué dans un deuxième aliquote de 5µl de NaCl 150mM. Les solutions ont ensuite été mélangées et appliquées sur les cellules pendant 1h. Le milieur de transfection a ensuite été enlevé et remplacé par du milieu de culture frais, avec ou sans T3 (1nM). Après 24h, les cellules ont été lysées, la solution de lyse centrifugée, et le surnageant utilisé pour doser l'activité luciférase.

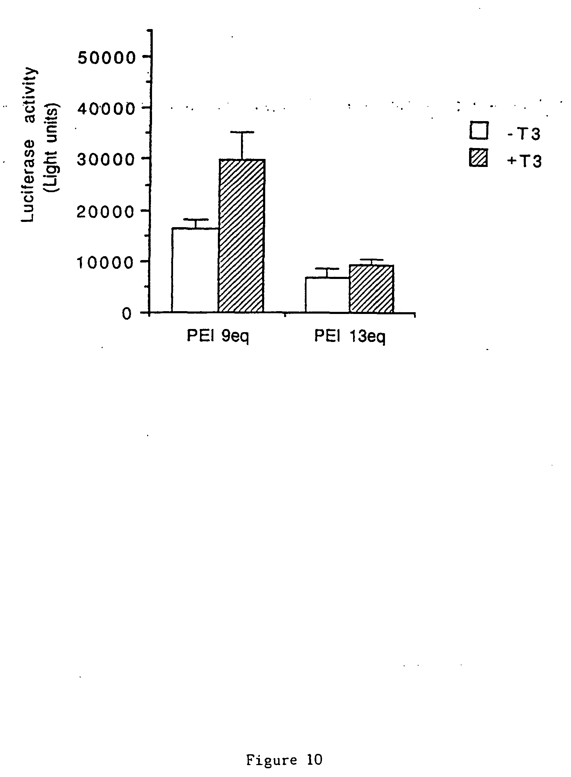

Les résultats obtenus sont présentés sur la figure 10. Ils montrent clairement que les compositions selon l'invention permettent de transférer de l'ADN dans les cellules neuronales, et que l'ADN ainsi transféré est fonctionnel puisque son activité est amplifiée en présence de T3. Dans l'expérience contrôle effectuée en présence du plasmide seul, aucune activité luciférase n'a été détectée.

### EXEMPLE 13 - Transfert d'oligonucléotides antisens dans une culture primaire de neurones embryonaires.

Les expériences ont été réalisées sur des cultures primaires de neurones préparées comme dans l'exemple 12. Après 36 h, les cultures ont été transfectées avec 2 µM d'oligonucléotide antisens 18mer (ODN) complexé avec 9 équivalents d'amines par rapport aux phosphates de PEI800K.

L'ODN utilisé est dirigé contre une région du gène codant pour le récepteur alpha des hormones thyroïdiennes. Sa séquence est la suivante :
5'-GCTGGGCTTCTGTTCCAT-3'

Pour 4 puits de 250µl de milieu de culture, les produits suivants ont été utilisés :
- 8 µl d'une solution d'ODN à 0,25mM dilués dans 10µl de NaCl 150mM, et,
- 20,8 µl d'une solution aqueuse de PEI800K 12 mM, pH7, préalablement dilués dans un deuxième aliquot de 51,2 µl de NaCl 150mM.

Les solutions ont ensuite été mélangées et 20µl du mélange ont été appliqués sur les cellules pendant 45min. Le milieu de transfection a ensuite été enlevé et remplacé par du milieu de culture frais. Les cellules ont ensuite été fixées dans une solution de paraformaldéhyde 4%, rincées, montées dans du Mowiol, et examinées au microscope à fluorescence.

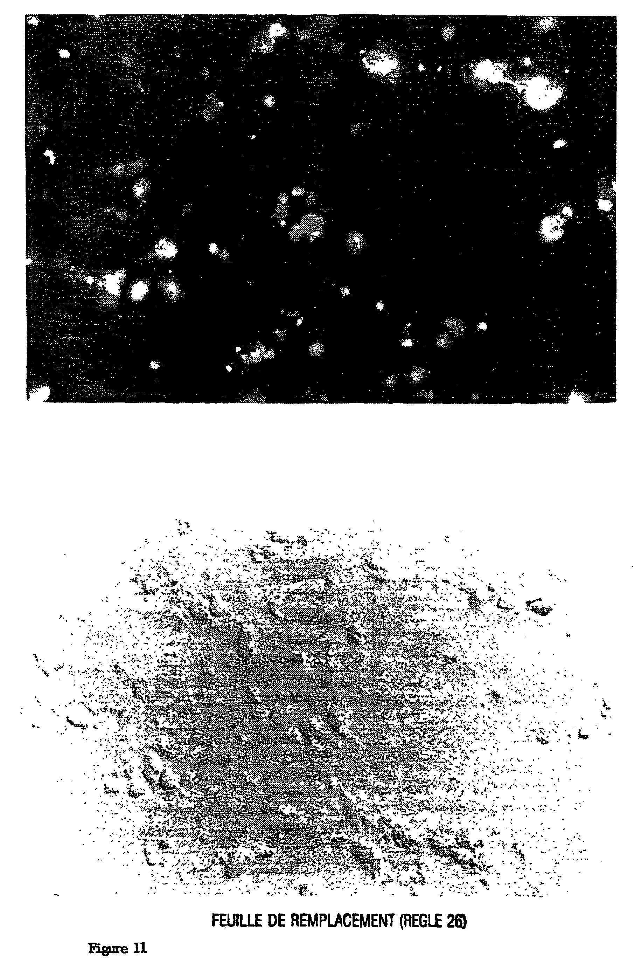

Les résultats obtenus sont présentés sur la figure 11. Ils montrent clairement que les compositions selon l'invention permettent de transférer des oligonucléotides antisens dans les cellules neuronales. Dans l'expérience contrôle effectuée en présence de l'oligonucléotide seul, aucune fluorescence n'a été détectée.

### EXEMPLE 14 - Utilisation des compositions de l'invention pour le transfert d'acides nucléiques in vivo, dans le cerveau de souris nouveau-nées.

Cet exemple décrit le transfert du plasmide pCMV-Luc in vivo, dans le cerveau - de souris nouveau-nées. Il démontre les propriétés particulièrement avantageuses des compositions selon l'invention, notamment pour des applications de thérapie génique.

30 µg de plasmide pCMV-luc (7,5 µl d'une solution stock à 4µg/µl) ont été dilués dans 22,5 µl de glucose 5% stérile (concentration finale de 1 µg/µl). Ensuite, 8,5 µl de PEI800K 100 mM (préparé dans l'eau et tamponé à pH 6,9) ont été ajoutés. La composition ainsi obtenue contient donc 9 équivalents d'aminés par rapport aux phosphates.

Le mélange a été vortexé rapidement et utilisé pour des injections intracérébrales chez des souris nouveau-nées. Pour cela, les souris ont été anesthésiées par le froid (placées sur une feuille d'aluminium en contact avec de la glace), puis 2 µl de mélange (2 µg d'acide nucléique) ont été injectés par souris. Les injections ont été réalisées dans le cortex, à l'aide d'un micromanipulateur et une microseringue reliées à une microélectrode.

Les cerveaux ont été prélevés 48 heures plus tard, homogénéisés, centrifugés et le surnageant utilisé pour le dosage de la luciférase. Pour cela, le surnageant a été incubé en présence d'un tampon comprenant de la luciférine, du coenzyme A et de l'ATP, et la lumière émise (généralement pendant 10 secondes) a été mesurée avec un luminomètre (Wood K. (1990) Promega Notes, 28).

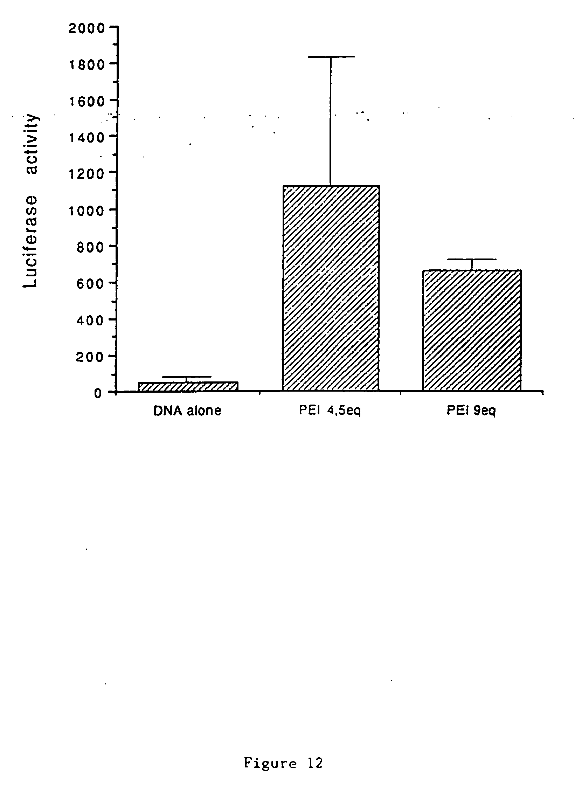

Les résultats obtenus sont présentés sur la figure 12. Ils montrent clairement que les compositions permettent de transférer efficacement le plasmide dans le cervau des souris alors qu'aucune activité luciférase significative n'est observée lorsque le transfert est réalisé au moyen du plasmide seul.

## Revendications

1. Composition constituée d'une solution d'un acide nucléique et d'une solution d'un polymère cationique réunies et homogénéisées, le polymère cationique étant de formule générale (I) : 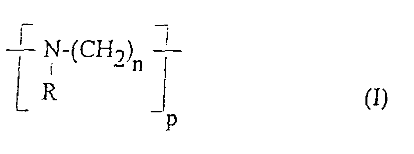 dans laquelle
- R peut être un atome d'hydrogène ou un groupe de formule 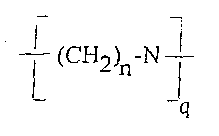
- n est un nombre entier compris entre 2 et 10;
- p et q sont des nombres entiers,
étant entendu que la somme p+q est telle que le poids moléculaire moyen du polymère soit compris entre 100 et 10⁷ Da, et
- les proportions respectives du polymère et de l'acide nucléique sont choisies de sorte que le rapport R amines du polymère phosphates de l'acide nucléique soit compris entre 5 et 30.

2. Composition selon la revendication 1 **caractérisée en ce que**, dans la formule (1), n est compris entre 2 et 5.

3. Composition selon les revendications 1 ou 2 **caractérisée en ce que** le polymère possède un poids moléculaire moyen compris entre 10³ et 5.10⁶ DA.

4. Composition selon l'une des revendications 1 à 3 **caractérisée en ce que** le polymère est choisi parmi le polyéthylène imine (PEI) et polypropylène imine (PPI).

5. Composition selon la revendication 4 **caractérisée en ce que** le polymère est choisi parmi le polyéthylène imine de poids moléculaire moyen 50 000 (PEI50K) et le polyéthylène imine de poids moléculaire moyen 800 000 (PEI800K).

6. Composition selon la revendication 5 **caractérisée en ce que** le rapport R est compris entre 5 et 15.

7. Compositions selon l'une des revendications précédentes **caractérisée en ce qu'**elle comprend en plus un ou plusieurs adjuvants capables de s'associer au complexe polymère/acide nucléique et d'améliorer le pouvoir transfectant, ledit adjuvant étant choisi parmi les lipides, protéines, lipopolyamines et polymères synthétiques.

8. Composition selon la revendication 7 **caractérisée en ce que** l'adjuvant est un lipide cationique.

9. Composition selon la revendication 8 **caractérisée en ce que** le lipide cationique est une ou plusieurs lipopolyamines.

10. Composition selon la revendication 9 **caractérisée en ce que** la lipopolyamine répond à la formule générale H₂N-(-(CH)ₘ-NH-)1-H dans laquelle m est un nombre entier compris entre 2 et 6 et 1 est un nombre entier compris entre 1 et 5, m pouvant varier entre les différents groupes de carbone compris entre deux amines.

11. Composition selon la revendication 10 **caractérisée en ce que** la lipopolyamine est choisie parmi la dioctadécylamidoglycyl spermine (DOGS) ou la 5-carboxyspermylamide de la palmitoylphosphatidylethanolamine (DPPES).

12. Composition selon la revendication 7 **caractérisée en ce que** l'adjuvant est un ou plusieurs lipides neutres.

13. Composition selon la revendication 12 **caractérisée en ce que** le ou les lipides neutres sont choisis parmi les lipides synthétiques ou naturels, zwitterioniques ou dépourvus de charge ionique dans les conditions physiologiques.

14. Composition selon la revendication 13 **caractérisée en ce que** le ou les lipides neutres sont des lipides à 2 chaînes grasses.

15. Composition selon la revendication 13 **caractérisée en ce que** le ou les lipides neutres sont choisis parmi la dioléoylphosphatidyléthanolamine (DOPE), l'oléoyl-palmitoylphos-phatidyléthanolamine (POPE), le di-stéaroyl, -palmitoyl,-mirystoyl phosphatidyléthanolamine ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines) et les asialogangliosides (tels que notamment les asialoGM 1 et GM2).

16. Composition selon la revendication 1 **caractérisée en ce que** l'acide nucléique est un acide désoxyribonucléique.

17. Composition selon la revendication 1 **caractérisée en ce que** l'acide nucléique est un acide ribonucléique.

18. Composition selon la revendication 16 ou 17 **caractérisée en ce que** l'acide nucléique est modifié chimiquement.

19. Composition selon la revendication 16 à 18 **caractérisée en ce que** l'acide nucléique est un antisens.

20. Composition selon l'une des revendications 16 à 19 **caractérisée en ce que** l'acide nucléique comporte un gène thérapeutique.

21. Composition selon l'une des revendications 16 à 19 **caractérisée en ce que** l'acide nucléique code pour une protéine antigénique.

22. Composition selon la revendication 21 **caractérisée en ce que** l'acide nucléique code pour une protéine virale.

23. Composition selon la revendication 7 **caractérisée en ce qu'**elle comprend de 0,1 à 20 équivalents molaires d'adjuvant pour 1 équivalent molaire de phosphates de l'acide nucléique, et, plus préférentiellement, de 1 à 5.

24. Composition selon l'une quelconque des revendications précédentes **caractérisée en ce qu'**elle comprend en plus un élément de ciblage constitué par un sucre et/ou un peptide, tel que un anticorps ou fragment d'anticorps, un ligand de récepteur cellulaire ou un fragment de celui-ci, ou un récepteur ou fragment de récepteur.

25. Composition selon la revendication 22 ou 25 **caractérisée en ce que** l'élément de ciblage est lié de manière covalent au polymère de formule (I).

26. Composition selon l'une quelconque des revendications précédentes **caractérisée en ce qu'**elle comprend en plus un véhicule pharmaceutiquement acceptable pour une formulation injectable.

27. Composition selon l'une quelconque des revendications précédentes **caractérisée en ce qu'**elle comprend un véhicule pharmaceutiquement acceptable pour une application sur la peau et/ou les muqueuses.

28. Composition telle que définie dans l'une quelconque des revendications 1 à 27 pour utilisation en tant que médicament.

29. Composition telle que définie dans l'une quelconque des revendications 1 à 27 pour utilisation en tant que vaccin.

30. Utilisation d'un polymère cationique tel que défini dans la revendication 1 pour le transfert *in vitro* d'acides nucléiques dans les cellules.

## Claims

1. Composition consisting of a solution of a nucleic acid and a solution of a cationic polymer, which are combined and homogenized, the cationic polymer being of general formula (I): 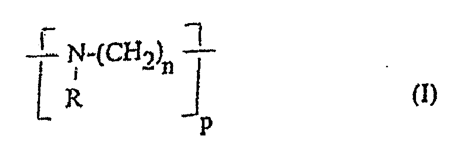 in which:
- R may be a hydrogen atom or a group of formula: 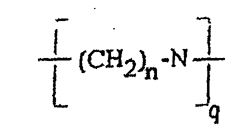
- n is an integer between 2 and 10;
- p and q are integers,
it being understood that the sum p+q is such that the average molecular weight of the polymer is between 100 and 10⁷ Da, and
- the respective proportions of the polymer and of the nucleic acid are chosen such that the polymer amines/nucleic acid phosphates ratio R is between 5 and 30.

2. Composition according to Claim 1, **characterized in that**, in formula (I), n is between 2 and 5.

3. Composition according to Claim 1 or 2, **characterized in that** the polymer has an average molecular weight of between 10³ and 5 × 10⁶ Da.

4. Composition according to one of-Claims 1 to 3, **characterized in that** the polymer is chosen from polyethyleneimine (PEI) and polypropyleneimine (PPI).

5. Composition according to Claim 4, **characterized in that** the polymer is chosen from polyethyleneimine with an average molecular weight of 50 000 (PEI50K) and polyethyleneimine with an average molecular weight of 800 000 (PEI800K).

6. Composition according to Claim 5, **characterized in that** the ratio R is between 5 and 15.

7. Composition according to one of the preceding claims, **characterized in that** it also comprises one or more adjuvants capable of associating with the polymer/nucleic acid complex and of improving the transfecting capacity, said adjuvant being chosen from lipids, proteins, lipopolyamines and synthetic polymers.

8. Composition according to Claim 7, **characterized in that** the adjuvant is a cationic lipid.

9. Composition according to Claim 8, **characterized in that** the cationic lipid is one or more lipopolyamines.

10. Composition according to Claim 9, **characterized in that** the lipopolyamine corresponds to the general formula H₂N-(-(CH)ₘ-NH-)₁-H in which m is an integer between 2 and 6 and 1 is an integer between 1 and 5, it being possible for m to vary between the various carbon groups between two amines.

11. Composition according to Claim 10, **characterized in that** the lipopolyamine is chosen from dioctadecylamidoglycyl spermine (DOGS) or palmitoylphosphatidylethanolamine 5-carboxyspermylamide (DPPES).

12. Composition according to Claim 7, **characterized in that** the adjuvant is one or more neutral lipids.

13. Composition according to Claim 12, **characterized in that** the neutral lipid(s) is (are) chosen from synthetic or natural lipids which are zwitterionic or devoid of ionic charge under physiological conditions.

14. Composition according to Claim 13, **characterized in that** the neutral lipid(s) is (are) lipids comprising two fatty chains.

15. Composition according to Claim 13, **characterized in that** the neutral lipid(s) is (are) chosen from dioleoylphosphatidylethanolamine (DOPE), palmitoyloleoylphosphatidylethanolamine (POPE), distearoyl-, dipalmitoyl- or dimyristoylphosphatidylethanolamine, and also derivatives thereof which are N-methylated 1 to 3 times; phosphatidylglycerols, diacylglycerols, glycosyldiacylglycerols, cerebrosides (such as, in particular, galactocerebrosides), sphingolipids (such as, in particular, sphingomyelins) and asialogangliosides (such as, in particular, asialoGM1 and GM2).

16. Composition according to Claim 1, **characterized in that** the nucleic acid is a deoxyribonucleic acid.

17. Composition according to Claim 1, **characterized in that** the nucleic acid is a ribonucleic acid.

18. Composition according to Claim 16 or 17, **characterized in that** the nucleic acid is chemically modified.

19. Composition according to Claims 16 to 18, **characterized in that** the nucleic acid is an antisense nucleic acid.

20. Composition according to one of Claims 16 to 19, **characterized in that** the nucleic acid contains a therapeutic gene.

21. Composition according to one of Claims 16 to 19, **characterized in that** the nucleic acid encodes an antigenic protein.

22. Composition according to Claim 21, **characterized in that** the nucleic acid encodes a viral protein.

23. Composition according to Claim 7, **characterized in that** it comprises from 0.1 to 20 molar equivalents of adjuvant per molar equivalent of nucleic acid phosphates, and preferably from 1 to 5.

24. Composition according to any one of the preceding claims, **characterized in that** it also comprises a targeting element consisting of a sugar and/or a peptide, such as an antibody or antibody fragment, a cell receptor ligand or a fragment thereof, or a receptor or receptor fragment.

25. Composition according to Claim 22 or 25, **characterized in that** the targeting element is covalently bonded to the polymer of formula (I).

26. Composition according to any one of the preceding claims, **characterized in that** it also comprises a carrier that is pharmaceutically acceptable for an injectable formulation.

27. Composition according to any one of the preceding claims, **characterized in that** it comprises a pharmaceutical carrier that is acceptable for application to the skin and/or the mucous membranes.

28. Composition as defined in any one of Claims 1 to 27, for use as a medicament.

29. Composition as defined in any one of Claims 1 to 27, for use as a vaccine.

30. Use of a cationic polymer as defined in Claim 1 for the in vitro transfer of nucleic acids into cells.

## Patentansprüche

1. Zusammensetzung, gebildet aus einer Lösung einer Nukleinsäure und einer Lösung eines kationischen Polymers, die vereinigt und homogenisiert worden sind, wobei das kationische Polymer die allgemeine Formel (I) aufweist: 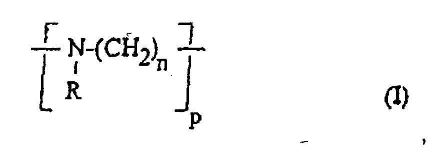 in welcher:
- R ein Wasserstoffatom oder eine Gruppe der Formel 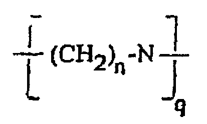 sein kann;
- n eine ganze Zahl zwischen 2 und 10 eingeschlossen ist;
- p und q ganze Zahlen sind,
mit der Maßgabe, dass die Summe p+q derart ist, dass das mittlere Molekulargewicht des Polymers zwischen 100 und 10⁷ Da eingeschlossen liegt, und
- wobei die jeweiligen Anteile des Polymers und der Nukleinsäure derart ausgewählt werden, dass das Verhältnis R der Amingruppen des Polymers zu den Phosphatgruppen der Nukleinsäure zwischen 5 und 30 eingeschlossen liegt.

2. Zusammensetzung nach Anspruch 1, **dadurch gekennzeichnet, dass** in der Formel (I) n zwischen 2 und 5 eingeschlossen ist.

3. Zusammensetzung nach den Ansprüchen 1 oder 2, **dadurch gekennzeichnet, dass** das Polymer ein mittleres Molekulargewicht zwischen 10³ und 5.10⁶ Da eingeschlossen aufweist.

4. Zusammensetzung nach einem der Ansprüche 1 bis 3, **dadurch gekennzeichnet, dass** das Polymer unter Polyethylenimin (PEI) und Polypropylenimin (PPI) ausgewählt wird.

5. Zusammensetzung nach Anspruch 4, **dadurch gekennzeichnet, dass** das Polymer unter Polyethylenimin mit einem mittleren Molekulargewicht von 50000 (PE150K) und Polyethylenimin mit einem mittleren Molekulargewicht von 800000 (PEI800K) ausgewählt wird.

6. Zusammensetzung nach Anspruch 5, **dadurch gekennzeichnet, dass** das Verhältnis R zwischen 5 und 15 eingeschlossen liegt.

7. Zusammensetzungen nach einem der vorangegangenen Ansprüche, **dadurch gekennzeichnet, dass** sie außerdem einen oder mehrere Hilfsstoffe umfasst, die in der Lage sind, sich mit dem Polymer/Nukleinsäure-Komplex zu assoziieren und das Transfektionsvermögen zu erhöhen, wobei der Hilfsstoff unter den Lipiden, Proteinen, Lipopolyaminen und synthetischen Polymeren ausgewählt wird.

8. Zusammensetzung nach Anspruch 7, **dadurch gekennzeichnet, dass** der Hilfsstoff ein kationisches Lipid ist.

9. Zusammensetzung nach Anspruch 8, **dadurch gekennzeichnet, dass** das kationische Lipid ein oder-mehrere Lipopolyamine ist.

10. Zusammensetzung nach Anspruch 9, **dadurch gekennzeichnet, dass** das Lipopolyamin der allgemeinen Formel H₂N-(-(CH)ₘ-NH-)1-H entspricht, in weicher m eine ganze Zahl zwischen 2 und 6 eingeschlossen ist und I eine ganze Zahl zwischen 1 und 5 eingeschlossen ist, wobei m zwischen den verschiedenen Kohlenstoff-Gruppen, die zwischen zwei Aminen enthalten sind, variieren kann.

11. Zusammensetzung nach Anspruch 10, **dadurch gekennzeichnet, dass** das Lipopolyamin unter Dioctadecylamidoglycylspermin (DOGS) oder 5-Carboxyspermylamid von Palmitoylphosphatidylethanolamin (DPPES) ausgewählt wird.

12. Zusammensetzung nach Anspruch 7, **dadurch gekennzeichnet, dass** der Hilfsstoff ein oder mehrere neutrale Lipide ist.

13. Zusammensetzung nach Anspruch 12, **dadurch gekennzeichnet, dass** das oder die neutralen Lipide ausgewählt werden unter den synthetischen oder natürlichen Lipiden, die unter physiologischen Bedingungen zwitterionisch oder frei von Ionenladung sind.

14. Zusammensetzung nach Anspruch 13, **dadurch gekennzeichnet, dass** das oder die neutralen Lipide Lipide mit 2 Fettketten sind.

15. Zusammensetzung nach Anspruch 13, **dadurch gekennzeichnet, dass** das oder die neutralen Lipide ausgewählt werden unter Dioleoylphosphatidylethanolamin (DOPE), Oleoylpalmitoylphosphatidylethanolamin (POPE), Distearoyl-, -palmitoyl-, -myristoylphosphatidylethanolamin wie auch deren 1- bis 3-fach N-methylierten Derivaten; den Phosphatidylglycerolen, den Diacylglycerolen, den Glycosyldiacylglycerolen, den Cerebrosiden (wie insbesondere den Galactocerebrosiden), den Sphingolipiden (wie insbesondere den Sphingomyelinen) und den Asialogangliosiden (wie insbesondere den AsialoGM1 und GM2).

16. Zusammensetzung nach Anspruch 1, **dadurch gekennzeichnet, dass** die Nukleinsäure eine Desoxyribonukleinsäure ist.

17. Zusammensetzung nach Anspruch 1, **dadurch gekennzeichnet, dass** die Nukleinsäure eine Ribonukleinsäure ist.

18. Zusammensetzung nach Anspruch 16 oder 17, **dadurch gekennzeichnet, dass** die Nukleinsäure chemisch modifiziert ist.

19. Zusammensetzung nach Anspruch 16 bis 18, **dadurch gekennzeichnet, dass** die Nukleinsäure eine Antisense-Nukleinsäure ist.

20. Zusammensetzung nach einem der Ansprüche 16 bis 19, **dadurch gekennzeichnet, dass** die Nukleinsäure ein therapeutisches Gen umfasst.

21. Zusammensetzung nach einem der Ansprüche 16 bis 19, **dadurch gekennzeichnet, dass** die Nukleinsäure ein antigenes Protein kodiert.

22. Zusammensetzung nach Anspruch 21, **dadurch gekennzeichnet, dass** die Nukleinsäure ein virales Protein kodiert.

23. Zusammensetzung nach Anspruch 7, **dadurch gekennzeichnet, dass** sie 0,1 bis 20 Mol-äquivalente Hilfsstoff auf 1 Moläquivalent Phosphatgruppen der Nukleinsäure und mehr bevorzugt 1 bis 5 umfasst.

24. Zusammensetzung nach einem der vorangegangenen Ansprüche, **dadurch gekennzeichnet, dass** sie außerdem ein Targeting-Element, das aus einem Zucker und/oder einem Peptid gebildet wird, wie einen Antikörper oder Antikörperfragment, einen Ligand eines zellulären Rezeptors oder ein Fragment eines solchen oder einen Rezeptor oder ein Rezeptorfragment, umfasst.

25. Zusammensetzung nach Anspruch 22 oder 25, **dadurch gekennzeichnet, dass** das Targeting-Element auf kovalente Weise mit dem Polymer der Formel (I) verknüpft ist.

26. Zusammensetzung nach einem der vorangegangenen Ansprüche, **dadurch gekennzeichnet, dass** sie außerdem einen pharmazeutisch annehmbaren Träger für eine injizierbare Formulierung umfasst.

27. Zusammensetzung nach einem der vorangegangenen Ansprüche, **dadurch gekennzeichnet, dass** sie einen pharmazeutisch annehmbaren Träger für ein Auftragen auf die Haut und/oder die Schleimhäute umfasst.

28. Zusammensetzung, wie in einem der Ansprüche 1 bis 27 definiert, für eine Verwendung als Arzneimittel.

29. Zusammensetzung, wie in einem der Ansprüche 1 bis 27 definiert, für eine Verwendung als Impfstoff.

30. Verwendung eines kationischen Polymers, wie in Anspruch 1 definiert, für den *in vitro-*Transfer von Nukleinsäuren in Zellen.
